# EUROPÄISCHE PATENTSCHRIFT

(11) **EP 1 155 776 B1**
(45) Veröffentlichungstag und Bekanntmachung des Hinweises auf die Patenterteilung: **06.09.2006**
(21) Anmeldenummer: 01111758.7
(22) Anmeldetag: 15.05.2001
(51) Int. Cl.: B24B 27/027, B24B 45/00, B24B 19/14, B24B 49/12, B23B 31/107, B23Q 17/24, A61B 17/32, A61C 1/14

(54) **Austauschbarer Werkzeugeinsatz für ein endoskopisches Bearbeitungsgerät und derartiges endoskopisches Bearbeitungsgerät**
Removable tool insert for endoscopic processing apparatus and such an endoscopic processing apparatus
Elément d'outil amovible pour dispositif d'usinage endoscopique ainsi qu'un tel dispositif d'usinage endoscopique

(30) Priorität: 16.05.2000 DE 10023685; 14.09.2000 EP 00119988
(43) Veröffentlichungstag der Anmeldung: 21.11.2001
(73) Patentinhaber: Storz-Endoskop GmbH, 8200 Schaffhausen (CH)
(72) Erfinder: Rüegg, Thomas, Toronto, Ontario M4S 3G9 (CA); Krattiger, Beat, CH-8222 Beringen (CH); Haan, Harald, CH-8200 Schaffhausen (CH)
(74) Vertreter: Heuckeroth, Volker

(56) Entgegenhaltungen:
- DE-C- 919 905
- US-A- 5 349 940

## Beschreibung

Die Erfindung betrifft einen austauschbaren Werkzeugeinsatz für ein endoskopisches Bearbeitungsgerät, mit einer Welle, die an ihrem distalen Ende mit einer Werkzeugeinheit und an ihrem proximalen Ende mit einem Kupplungselement zur lösbaren Verbindung mit einem Motor verbunden ist, wobei die Welle flexibel als Litze oder als Profil in Form eines Drahtes, Rohrs oder kantigen Profils ausgebildet ist, und mit einem Verriegelungsmechanismus zum lösbaren Halten des Werkzeugeinsatzes am Bearbeitungsgerät.

Die Erfindung betrifft ferner ein mit einem derartigen Werkzeugeinsatz versehenes endoskopisches Bearbeitungsgerät.

Ein Werkzeugeinsatz und ein Bearbeitungsgerät der zuvor genannten Art ist aus der US 5,349,940 bekannt.

Ein solches endoskopisches Bearbeitungsgerät kann für technische Anwendungen, auf medizinischem Gebiet, insbesondere auf dem Gebiet der minimal-invasiven Chirurgie, und auch in der Zahnmedizin oder Zahntechnik verwendet werden.

Bei technischen Anwendungen beinhaltet eine mit dem Bearbeitungsgerät durchgeführte Bearbeitung beispielsweise das Entgraten von Gußteilen, das Ausschleifen von Einschlagkerben an Triebwerksschaufeln, das Abschleifen von Korrosion zur Oberflächenanalyse, beispielsweise bei Rißprüfungen, das Abschleifen von Schweißnähten, das Bohren von Löchern etc. Im Rahmen der minimal-invasiven Chirurgie kann ein solches Bearbeitungsgerät dazu benutzt werden, durch eine kleine Inzision in der Körperdecke Gewebe im Körperinneren zu behandeln, beispielsweise abzutragen.

Allgemein dient ein derartiges endoskopisches Bearbeitungsgerät zur universellen endoskopischen Bearbeitung und/oder Analyse unter optischer Sichtkontrolle mittels eines rotierenden Werkzeugs an schwer zugänglichen Stellen.

Bei bekannten starren Bearbeitungsgeräten erfolgt die Bewegungsübertragung vom proximal gelegenen Motor zur distalen Werkzeugeinheit mittels starren Antriebswellen oder Transmissionsriemen. Derartige Übertragungselemente erfordern Getriebeeinheiten oder Umlenkrollen, die Kosten und einen Platz- und Effizienzverlust bewirken. Die Konstruktion der Energietransmission vom proximal gelegenen Motor zur distalen Werkzeugeinheit ist kompliziert und störungsanfällig. Starre Antriebswellen, die parallel und nahe der Längsachse des Arbeitsschafts des Bearbeitungsgerätes liegen, können nicht direkt an einen Motor angekuppelt werden, da der Motor gewisse Mindestabmessungen aufweist, die mit der starren Linsenoptik des endoskopischen Bearbeitungsgerätes kollidieren können. Dies könnte allerdings zu Lasten einer schlechteren Auflösung durch die Verwendung von Faseroptiken als Endoskopoptik behoben werden.

Fliehkräfte und Unwuchten, die beim Gebrauch des endoskopischen Bearbeitungsgerätes an der Werkzeugeinheit und dem Übertragungselement in Form der Welle auftreten können, führen zu Vibrationen, die die Drehzahl der Werkzeugeinheit nach oben begrenzen. Durch eine derartige konstruktionsbedingte Niedertourigkeit folgt jedoch nachteiligerweise eine geringere Abtragsleistung.

Des weiteren ist bei einem derartigen Bearbeitungsgerät nicht nur die distalseitige Werkzeugeinheit einem Verschleiß unterworfen, sondern auch die Welle und die Kupplung der Welle an dem Motor. Es sind Bearbeitungsgeräte bekannt, bei denen die Werkzeugeinheit mit der Welle und dem Kupplungselement nicht oder zumindest nicht einfach ausgetauscht werden kann, wodurch sich derartige Bearbeitungsgeräte als in der Wartung sehr kostspielig erweisen, wie beispielsweise bei dem aus der US 5,349,940 bekannten Werkzeugeinsatz.

Des weiteren sind Bearbeitungsgeräte für medizinische Anwendungen bekannt, die zur Schmierung der Lager mit Spülflüssigkeit betrieben werden müssen. Ein derartiges Bearbeitungsgerät ist daher nicht einsetzbar für technische Anwendungen. Dieses bekannte Bearbeitungsgerät weist zwar einen austauschbaren Werkzeugeinsatz auf, der alle Verschleißelemente wie Lager, Arbeitskopf sowie Welle und Wellenschutz enthält, der jedoch insgesamt starr ist und konstruktionsbedingt nicht für hohe Tourenzahlen ausgelegt ist.

Aus dem eingangs genannten Dokument US 5,349,940 ist ein endoskopisches Bearbeitungsgerät mit einem austauschbaren Werkzeugeinsatz bekannt, wobei der Werkzeugeinsatz eine flexible Welle aufweist, die an ihrem distalen Ende mit einer Werkzeugeinheit und an ihrem proximalen Ende mit einem Kupplungselement verbunden ist. Die Werkzeugeinheit, die den Arbeitskopf, beispielsweise einen Schleifkopf, aufweist, ist mit der flexiblen Welle über eine Steck-Schraubkupplung lösbar verbunden, so daß zum Austauschen der Werkzeugeinheit zuerst die Kupplung zwischen dieser und der Welle gelöst werden muß. Die Welle selbst ist axial ebenfalls mehrteilig ausgebildet und nur schwierig ausbaubar. Die Verbindung zwischen der Werkzeugeinheit, der Welle und dem Motor ist somit insgesamt vielteilig aufgebaut, d.h. es müssen mehrere Einzelteile zusammengesteckt und miteinander verschraubt werden, wodurch sich das Austauschen dieses bekannten austauschbaren Werkzeugeinsatzes und damit die Wartung des Bearbeitungsgerätes als kompliziert erweist.

DE 919 905 C offenbart einen Winkelstückkopf für zahnärztliche Zwecke für Werkzeuge mit konischem Schaft, die über eine auslösbare, federbelastete Verriegelungsvorrichtung gehalten werden. Die Verriegelungsvorrichtung weist eine federnde Stange auf, die an ihrem distalen Ende in eine Nut am Werkzeugeinsatz angreift. Die federnde Stange ist mit einem federbelasteten Druckknopf verbunden, mit dem die federnde Stange mit der Nut außer Eingriff gebracht werden kann. Die Verriegelungsvorrichtung dient zur Verriegelung eines Zahnbohrers mit starrem zylindrischem Schaft, der über einen Bohrhülsentrieb mit dem Antrieb des Bohrgerätes verbunden ist.

Der Erfindung liegt daher die Aufgabe zugrunde, einen eingangs genannten austauschbaren Werkzeugeinsatz für ein endoskopisches Bearbeitungsgerät bzw. ein derartiges Bearbeitungsgerät dahingehend weiterzubilden, daß der Werkzeugeinsatz einfach und schnell ausgetauscht werden kann, wobei alle verschleißbehafteten Teile mit geringem Manipulationsaufwand austauschbar sein sollen.

Erfindungsgemäß wird diese Aufgabe hinsichtlich des eingangs genannten austauschbaren Werkzeugeinsatzes durch die Merkmale des Anspruchs 1 gelöst, wobei der Verriegelungsmechanismus ein an der Werkzeugeinheit angeordnetes Verriegelungselement aufweist, in das ein Verriegelungsteil des Bearbeitungsgerätes eingreift, und daß die Welle mit der Werkzeugeinheit so verbunden ist, daß diese eine Einheit bilden, die bei Verriegelung des Verriegelungsmechanismus lösbar am Bearbeitungsgerät gehalten wird und nach Entriegelung des Verriegelungsmechanismus als Ganzes entnommen werden kann.

Die zuvor genannte Verbindung zwischen Werkzeugeinheit und Welle ist sowohl hinsichtlich des einfachen Austausches des austauschbaren Werkzeugeinsatzes vorteilhaft, weil diese beiden verschleißbehafteten Elemente, ohne miteinander verbunden oder getrennt werden zu müssen, gemeinsam und somit leicht handhabbar ausgetauscht werden können. Des weiteren ermöglicht es diese Ausgestaltung vorteilhafterweise, die Werkzeugeinheit und die Welle miniaturisiert auszugestalten, wodurch sehr hohe Drehzahlen des Werkzeugeinsatzes ermöglicht werden, ohne daß vibrationshemmende Maßnahmen wie dynamische Balancierung, Dämpfung und reibungshemmende Maßnahmen zwingend erforderlich sind. Eine derartige Miniaturisierung ist bei dem aus der US 5,349,940 bekanntem austauschbaren Werkzeugeinsatz wegen der Vielteiligkeit des Werkzeugeinsatzes nicht möglich. Der Verriegelungsmechanismus ist erfindungsgemäß so ausgebildet, daß die Verriegelung des Werkzeugeinsatzes, und zwar die Einheit aus Werkzeugeinheit und Welle, durch ein an der Werkzeugeinheit angeordnetes Verriegelungselement erfolgt, in das ein Verriegelungsteil des Bearbeitungsgerätes eingreift. Über die Werkzeugeinheit ist dann auch die Welle axial am Bearbeitungsgerät festgelegt.

Die Verbindung zwischen der Welle und der Werkzeugeinheit kann stoffschlüssig, beispielsweise durch Löten oder Schweißen, bewerkstelligt sein, oder die Werkzeugeinheit kann mit der Welle einstückig verbunden sein.

Die Welle ist als Litze, oder als Profil in Form eines Drahtes, Rohrs oder kantigen Profils gebildet.

Diese Maßnahmen stellen vorteilhafte Ausgestaltungen der Welle dar, um dieser eine entsprechende Flexibilität zu verleihen. Mit einer flexiblen Welle läßt sich der Werkzeugeinsatz auch in solchen Bearbeitungsgeräten verwenden, die einen flexiblen Schaft aufweisen, um schwer zugängliche Arbeitsbereiche zu erreichen. Bei einer Ausgestaltung der Welle als Hohlprofil kann dieses Hohlprofil auch zum Material-, Signal- bzw. Informationstransport zum Arbeitskopf der Werkzeugeinheit und von dieser weg genutzt werden.

In einer bevorzugten Ausgestaltung ist die Welle auch mit dem proximalen Kupplungselement so verbunden, daß das Kupplungselement zusammen mit der Welle und der Werkzeugeinheit nach Lösen des Verriegelungsmechanismus entnommen werden kann.

Durch diese Ausgestaltung sind sowohl die Werkzeugeinheit, die Welle als auch das Kupplungselement und somit alle verschleißbehafteten Teile auf einfach zu handhabende Weise leicht austauschbar, und zum Festlegen dieser integralen Einheit an dem Bearbeitungsgerät ist nur der zuvor beschriebene Verriegelungsmechanismus mit dem an der Werkzeugeinheit angeordneten Verriegelungselement und dem am Bearbeitungsgerät vorgesehenen Verriegelungsteil zu betätigen.

Der erfindungsgemäß vorgesehene Verriegelungsmechanismus, bei dem ein Verriegelungsteil des Bearbeitungsgerätes in ein Verriegelungselement der Werkzeugeinheit eingreift, stellt einen schnell lösbaren und schnell verriegelbaren Verriegelungsmechanismus dar, der das Austauschen des austauschbaren Werkzeugeinsatzes wesentlich vereinfacht. Wie bereits erwähnt, sind alle verschleißbehafteten Teile in einem austauschbaren Element zusammengefaßt, wodurch die Handhabung beim Austausch vereinfacht und außerdem auch die Kosten des austauschbaren Werkzeugeinsatzes hinsichtlich seines Herstellungsaufwandes verringert sind.

In einer weiteren bevorzugten Ausgestaltung weist die Werkzeugeinheit einen Arbeitskopf und einen Werkzeugschaft auf, und ist das Verriegelungselement als umlaufende Nut in dem Werkzeugschaft ausgebildet.

Beim Rotieren des Arbeitskopfes und des Werkzeugschaftes läuft das Verriegelungsteil in der Nut und sichert damit den austauschbaren Werkzeugeinsatz axial. Die Vorteile dieser Maßnahme bestehen in einem konstruktiv sehr einfachen Verriegelungsmechanismus, der sich durch einen einfachen Fertigungsschritt, nämlich dem Einbringen der umlaufenden Nut an dem Werkzeugschaft realisieren läßt. Ein derartiger Verriegelungsmechanismus hat den weiteren Vorteil, daß eine Verriegelung mit einem Minimalbedarf an zusätzlichem Schaftdurchmesser erzielt wird. Durch die Verriegelung des Werkzeugeinsatzes in seinem distalen Bereich, nämlich an der Werkzeugeinheit, wird bei der flexiblen Welle eine gute Werkzeugführung ermöglicht. Wäre der Werkzeugeinsatz proximal an der Motorkupplung verriegelt, würde durch die Längsfederwirkung der flexiblen Welle die Werkzeugeinheit bei axialer Anpreßkraft nach proximal ausweichen. Dies würde zu einer unpräzisen Werkzeughandhabe führen.

In einer weiteren bevorzugten Ausgestaltung ist eine Lagerbuchse vorgesehen, die einen Längsabschnitt des Werkzeugschafts aufnimmt und einen Schlitz aufweist, der so ausgebildet ist, daß das Verriegelungselement den Schlitz durchgreifen kann.

Diese Maßnahme hat den Vorteil, daß gleichzeitig das rotierende Werkzeug und die Lagerbuchse durch den Verriegelungsmechanismus verriegelt werden. Die Lagerbuchse kann bei der Entnahme des Werkzeugeinsatzes ebenfalls vom Bearbeitungsgerät entnommen und als verschleißbehaftetes Teil ausgetauscht werden. Wie später noch beschrieben wird, hat die Lagerbuchse ein geringes radiales Spiel.

In einer weiteren bevorzugten Ausgestaltung ist die Welle aus einer hochflexiblen Legierung gebildet.

Derartige hochflexible Legierungen können beispielsweise Nitinot® oder Tinel® sein.

In einer weiteren bevorzugten Ausgestaltung ist eine Spirale vorgesehen, die einen Längsabschnitt der Welle in der Spirale drehbar aufnimmt.

Dadurch, daß die Spirale die Welle umgibt, möglichst mit wenig Spiel, und dabei nicht mitrotiert, kann der Welle trotz ihrer Flexibilität eine stabilisierende Steifigkeit verliehen werden, und Umwuchten können sich nicht aufbauen.

Dabei ist es bevorzugt, wenn eine Ummantelung, vorzugsweise eine Kunststoffummantelung, vorgesehen ist, die die Spirale umgibt.

Wie bereits erwähnt, befindet sich zwischen der Spirale und der Welle möglichst wenig Spiel, so daß sich keine Unwucht aufbauen kann. Eine Unwucht würde neben den störenden Vibrationen auch zu Reibungsverlusten an der Innenseite der Spirale führen. Die Kunststoffummantelung dient vorteilhafterweise als Vibrationsdämpfer. Durch eine Dämpfung werden Resonanzen vernichtet, die durch Aufschaukelung der Vibrationen zu Reibungsverlusten führen können. Des weiteren schützt die Kunststoffummantelung vorteilhafterweise die Spirale vor Verformungen, die durch die Montage oder Demontage entstehen können. Darüber hinaus verhindert die Kunststoffummantelung einen Schmiermittelverlust.

Ein erfindungsgemäßes endoskopisches Bearbeitungsgerät mit den Merkmalen des Anspruchs 8 weist einen erfindungsgemäßen Werkzeugeinsatz gemäß einer oder mehreren zuvor genannten Ansprüche auf.

Dabei ist es bevorzugt, wenn sich das Verriegelungsteil senkrecht zur Längsachse des Bearbeitungsgerätes erstreckt und an seinem der Längsachse abgewandten Ende an einem parallel zur Längsachse verlaufenden Halteteil der Verriegelungseinheit angebracht ist.

Des weiteren ist es bevorzugt, wenn das Halteteil als im Wesentlichen senkrecht zur Längsachse federnde Lasche ausgebildet ist, so daß das Verriegelungsteil senkrecht zur Längsachse bewegbar ist.

Diese Ausgestaltung des Verriegelungsmechanismus mit dem Verriegelungsteil an dem als federnde Lasche ausgebildeten Halteteil hat den Vorteil, daß sowohl das Verriegeln als auch das Entriegeln sehr leicht zu handhaben ist, insbesondere beim Entriegeln· kommt das Verriegelungsteil selbsttätig mit dem Verriegelungselement an der Werkzeugeinheit außer Eingriff.

Dabei ist es weiter bevorzugt, wenn der Verriegelungsmechanismus eine in axialer Richtung verlagerbare Schiebehülse aufweist, die über die Lasche schiebbar ausgebildet ist und das Verriegelungsteil senkrecht zur Längsachse bewegt, so daß das Verriegelungsteil in Eingriff mit dem Verriegelungselement des Werkzeugeinsatzes bringbar ist.

Insgesamt hat der erfindungsgemäße Verriegelungsmechanismus den Vorteil, daß zum Austauschen des Werkzeugeinsatzes keine Schrauben angezogen oder gelöst werden müssen, sondern es muß lediglich die Schiebehülse zum Entriegeln nach proximal geschoben werden, wodurch die federnde Lasche mit dem daran angeordneten Verriegelungsteil selbsttätig nach außen federt, wodurch das Verriegelungsteil mit dem Verriegelungselement außer Eingriff kommt, und zum Verriegeln nach distal verschoben werden, wodurch die Schiebehülse die Lasche radial nach innen drückt und damit das Verriegelungsteil mit dem Verriegelungselement in Eingriff kommt.

In einer weiteren bevorzugten Ausgestaltung ist ein Einsatzkanal vorgesehen, in den der Werkzeugeinsatz in axialer Richtung ein- und ausführbar ist, wobei der Werkzeugeinsatz in dem Einsatzkanal mit Spiel aufgenommen ist.

Dieses Spiel hat die vorteilhafte Wirkung, daß sich Unwuchten des Werkzeugeinsatzes nicht stark auswirken können. Im Bereich der Lagerbuchse bewirkt diese Maßnahme, daß sich Unwuchten der Werkzeugeinheit nicht stark auswirken, sondern durch eine minimale Mitbewegung der Lagerbuchse kompensiert werden. Diese Selbstbalancierung verlängert die Lagerstandzeit, da die reibungsverursachenden Vibrationen minimiert werden. Im Bereich der Welle hat dieses Spiel ebenfalls den Vorteil, daß die Welle durch Unwuchten leicht mitbewegt wird, wodurch eine Selbstbalancierung eintritt, die die Vibrationen minimiert.

In einer weiteren bevorzugten Ausgestaltung weist das Bearbeitungsgerät einen Arbeitsschaft auf, in dem der Einsatzkanal und parallel zu diesem ein Kanal für ein Endoskop angeordnet ist.

In einer weiteren bevorzugten Ausgestaltung weist der Arbeitsschaft im Distalbereich ein flexibles Teil zum Auslenken der Werkzeugeinheit aus der Längsachse des Proximalbereichs des Arbeitsschafts auf.

Hierdurch können nun vorteilhafterweise schwer zugängliche Arbeitsbereiche endoskopisch bearbeitet bzw. analysiert werden und über die flexible Welle kann die Motordrehung durch die Biegung hindurch auf die Werkzeugeinheit übertragen werden.

Weitere Vorteile ergeben sich aus der nachfolgenden Beschreibung und der beigefügten Zeichnungen.

Ausführungsbeispiele der Erfindung sind in der Zeichnung dargestellt und werden mit Bezug auf diese hier näher beschrieben.

Es zeigen:
- Fig. 1a): eine Längsschnittdarstellung eines Arbeitsschaftes eines Bearbeitungsgerätes mit einem austauschbaren Werkzeugeinsatz, wobei der Werkzeugeinsatz am Bearbeitungsgerät verriegelt ist;
- Fig. 1b): eine Fig. 1a) ähnliche Darstellung, wobei der Werkzeugeinsatz entriegelt ist;
- Fig. 2: eine Seitenansicht des austauschbaren Werkzeugeinsatzes in Fig. 1, teilweise im Längsschnitt, in Alleinstellung in einem gekrümmten Zustand;
- Fig. 3: eine Seitenansicht eines endoskopischen Bearbeitungsgerätes mit einem starren Arbeitsschaft; und
- Fig. 4: eine Seitenansicht eines endoskopischen Bearbeitungsgerätes mit einem flexiblen Arbeitsschaft.

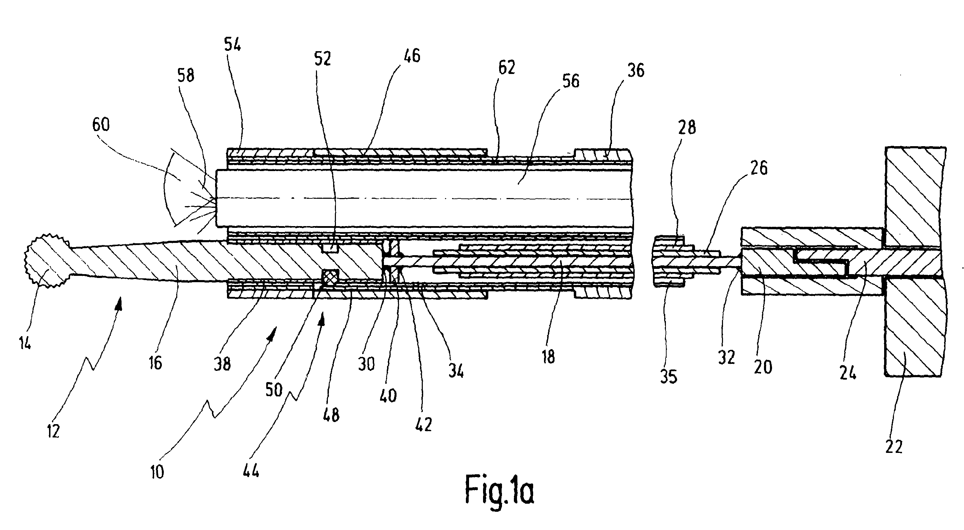

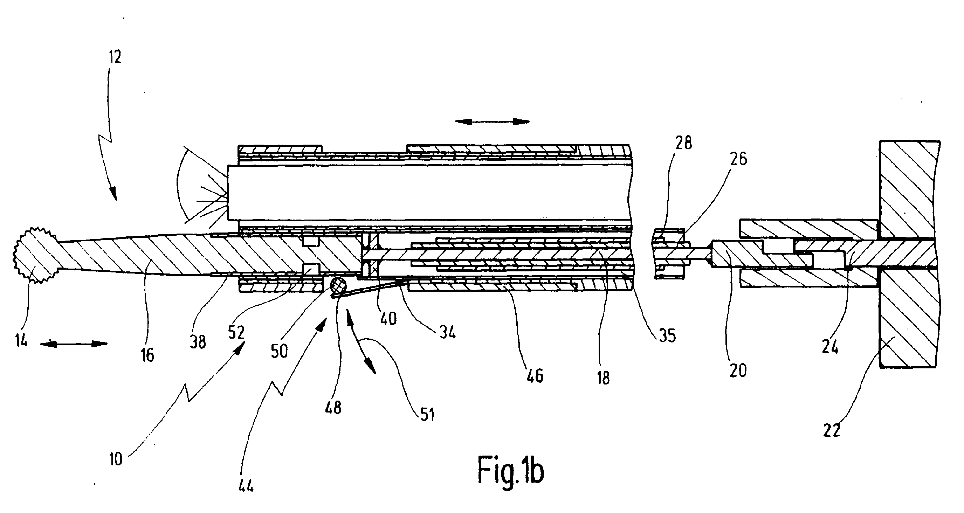

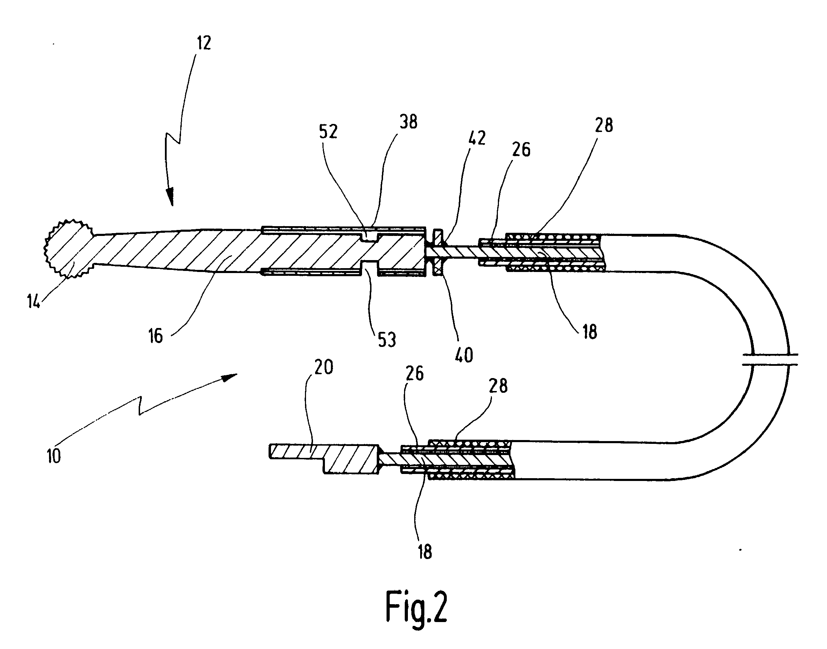

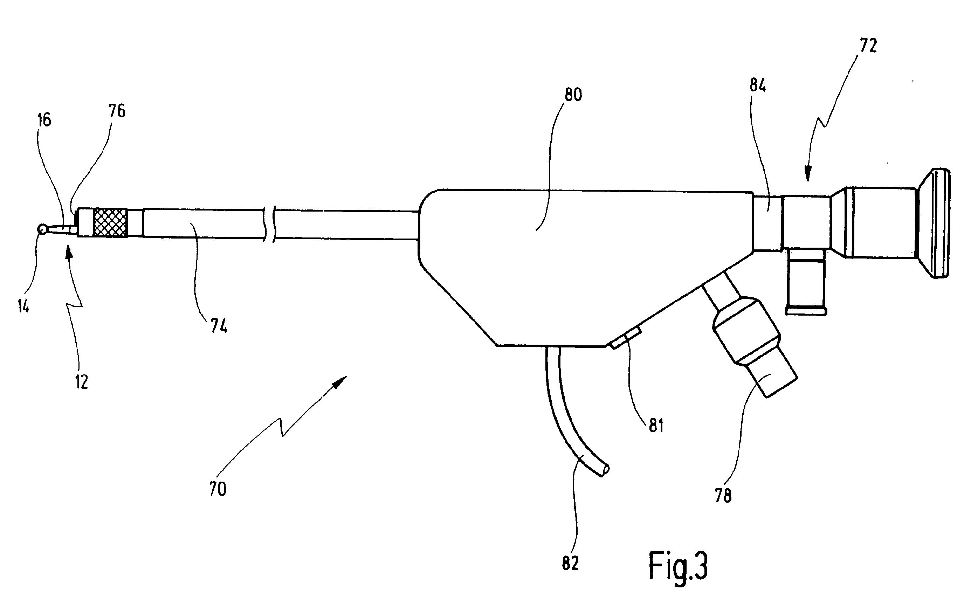

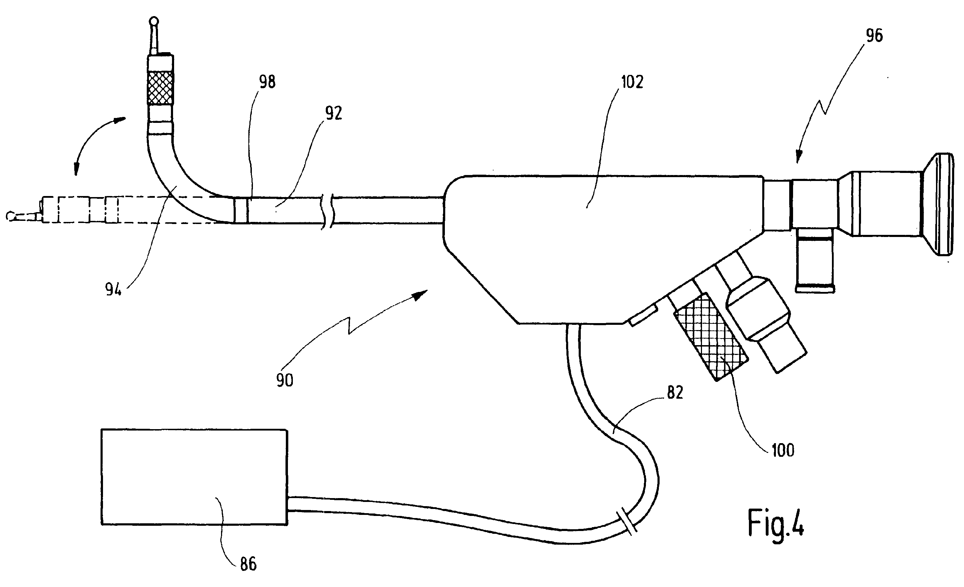

Mit Bezug auf Fig. 1 und 2 wird nachfolgend ein austauschbarer Werkzeugeinsatz 10 beschrieben, der in einem später zu beschreibenden endoskopischen Bearbeitungsgerät gemäß Fig. 3 und 4 verwendet wird. In Fig. 1 ist der austauschbare Werkzeugeinsatz 10 im in einem Bearbeitungsgerät eingesetzten Zustand, und in Fig. 2 in Alleinstellung und gegenüber Fig. 1 in einem gekrümmten Zustand dargestellt.

Der austauschbare Werkzeugeinsatz 10 und die später noch zu beschreibenden endoskopischen Bearbeitungsgeräte können sowohl in technischen Anwendungen, medizinischen Anwendungen, insbesondere der minimal-invasiv Chirurgie oder in der Zahnmedizin oder Zahntechnik verwendet werden.

Der kostengünstig herstellbare Werkzeugeinsatz 10 weist in einer realisierten Ausführung eine Werkzeugeinheit 12 auf, die einen Arbeitskopf 14, beispielsweise einen Schleifer, Fräser etc., sowie einen Werkzeugschaft 16 aufweist.

Am proximalen Ende des Werkzeugschaftes 16 ist eine Welle 18 in Form einer Litze aus Stahl mit der Werkzeugeinheit 12 zu einer Einheit verbunden, beispielsweise über eine Lötung 30. Die Welle 18 ist flexibel ausgebildet. Dabei ist die Welle 18 einerseits flexibler als ein massiver Draht, andererseits dämpft die Welle 18 Querschwingungen, die durch Unwuchten entstehen können.

Proximal ist die Werkzeugeinheit 12 über die Welle 18 mit einem Kupplungselement 20 zu einer Einheit verbunden, indem die Welle 18 mit ihrem proximalen Ende beispielsweise durch eine Lötung 32 mit dem Kupplungselement 20 verbunden ist. Das Kupplungselement 20 bildet die lösbare Verbindung des Werkzeugeinsatzes 10 mit einem Motor 22, genauer gesagt mit dessen Motorwelle 24.

Die Werkzeugeinheit 12, die Welle 18 und das Kupplungselement 20 bilden eine gemeinsam austauschbare Einheit, die beispielsweise auch insgesamt einstückig sein kann.

Die Welle 18 wird von einer nicht mitrotierenden Spirale 26 umgeben. Zwischen dieser Spirale 26 und der Welle 18 ist möglichst wenig Spiel, so daß sich keine Unwucht aufbauen kann. Eine Unwucht würde neben den störenden Vibrationen auch zu Reibungsverlusten an der Innenseite der Spirale 26 führen.

Die Spirale 26 ist ihrerseits durch eine Ummantelung, insbesondere Kunststoffummantelung 28 in Form eines Schrumpfschlauches ummantelt, die mehrere Funktionen hat. Erstens dient sie als Vibrationsdämpfer. Durch eine Dämpfung werden Resonanzen vernichtet, die durch Aufschaukelung der Vibrationen zu Reibungsverlusten führen können. Zweitens schützt sie die Spirale 26 vor Verformungen, die durch die Montage oder Demontage entstehen können. Drittens verhindert sie einen Schmiermittelverlust.

Der Werkzeugeinsatz 10 ist in einem Einsatzkanal 34 eines Bearbeitungsgerätes, wie es beispielsweise in Fig. 3 oder 4 dargestellt ist, angeordnet. In dem Einsatzkanal 34 ist der Werkzeugeinsatz 10 mit Spiel aufgenommen.

Der Werkzeugschaft 16 der Werkzeugeinheit 12 ist teilweise von einer Lagerbuchse 38 umgeben.

Proximalseitig des Werkzeugschaftes 16 ist eine Haltescheibe 40 an der Welle 18, beispielsweise über Lötungen 42, befestigt. Die Haltescheibe 40 dient als Sicherung gegen ein Wegrutschen der Lagerbuchse 38, was insbesondere beim Herausnehmen des Werkzeugeinsatzes 10 aus dem Bearbeitungsgerät wichtig ist, denn ohne die Haltescheibe 40 könnte die Lagerbuchse 38 im distalen Ende des Einsatzkanals 34 steckenbleiben.

Die Lagerbuchse 38 rotiert nicht mit.

Im distalen Bereich des Einsatzkanals 34 ist ein Verriegelungsmechanismus 44 vorgesehen, der die Lagerbuchse 38 und somit den gesamten austauschbaren Werkzeugeinsatz 10 verankert. Im gezeigten Ausführungsbeispiel weist der Verriegelungsmechanismus 44 im distalen Bereich des Bearbeitungsgerätes eine Schiebehülse 46 auf, die um den Arbeitsschaft 36 des Bearbeitungsgerätes herum angeordnet ist. Die Schiebehülse 46 kann in Längsrichtung nach distal, wie in Fig. 1a dargestellt ist, und nach proximal, wie in Fig. 1b dargestellt ist, verschoben werden. In der nach distal verschobenen Position (Fig. 1a) verriegelt die Schiebehülse 46 durch Einbiegen einer nach außen federnden Lasche 48, die inwärts mit einem Verriegelungsteil 50 versehen ist, die Werkzeugeinheit 12, an der ein Verriegelungselement 52 in Form einer umlaufenden Nut ausgebildet ist und damit die Welle 18 und das Kupplungselement 20 axial an dem Bearbeitungsgerät. Das Verriegelungsteil 50 ist in Form eines an der Lasche 48 befestigten Nocken ausgebildet, der in der distalen Position der Schiebehülse 46 in die das Verriegelungselement 52 bildende Nut eingreift, wodurch der Werkzeugeinsatz 10 am Bearbeitungsgerät axial festgelegt ist. Der Verriegelungsmechanismus 44 ermöglicht eine Verriegelung mit einem Minimalbedarf an zusätzlichem Schaftdurchmesser des Bearbeitungsgerätes. Dabei werden gleichzeitig die rotierende Werkzeugeinheit 12 und die Lagerbuchse 38 axial verriegelt, in der ein Schlitz 53 ausgebildet ist, durch den das Verriegelungsteil 50 durchdringt, um in die umlaufende Nut des Werkzeugschafts 16 zur Verriegelung einzugreifen.

Im nach proximal verschobenen Zustand der Schiebehülse 46 (Fig. 1b) federt die Lasche 48 radial nach außen und bringt damit das Verriegelungsteil 50 mit dem Verriegelungselement 52 außer Eingriff, wie mit einem Pfeil 51 angedeutet ist, wonach der Werkzeugeinsatz 10, d.h. die Einheit aus Werkzeugeinheit 12, Welle 18 und Kupplungselement 20 gemeinsam aus dem Einsatzkanal 34 entnommen werden kann. Die Haltescheibe 40 gewährleistet dabei, daß auch die Lagerbuchse 38 mit aus dem Einsatzkanal 34 herausgezogen wird.

Ein Abschlußstück 54 am distalen Ende des Arbeitsschafts 36 dient als distaler Anschlag für die Schiebehülse 46.

Durch die Verriegelung des Werkzeugeinsatzes 10 am distalen Ende des Bearbeitungsgerätes wird bei der flexiblen Welle 18 eine gute Werkzeugführung ermöglicht. Wäre der Werkzeugeinsatz 10 proximal am Kupplungselement 20 beispielsweise verriegelt, würde durch die Längsfederwirkung der flexiblen Welle 18 die Werkzeugeinheit 12 bei axialer Anpreßkraft nach proximal ausweichen. Dies würde zu einer unpräzisen Werkzeughandhabung führen.

Die Lagerbuchse 38 hat im Distalbereich des Bearbeitungsgerätes etwas Spiel, was bewirkt, daß sich Unwuchten der Werkzeugeinheit 12 nicht stark auswirken, sondern durch eine minimale Mitbewegung der Lagerbuchse 38 kompensiert werden. Diese Selbstbalancierung verlängert die Lagerstandzeit, da die reibungsverursachenden Vibrationen minimiert werden.

Ein ähnlicher Effekt tritt bei der Welle 18, d.h. dem Übertragungselement, auf. Die Spirale 26 wird durch Unwuchten der Welle 18 leicht mitbewegt. Die Bewegung wird durch das Spiel zwischen dem Einsatzkanal 34 und der Kunststoffummantelung 28 um die Spirale 26 ermöglicht. Durch die leichte Bewegung, die durch die Kunststoffummantelung 28 gedämpft wird, tritt eine Selbstbalancierung ein, die die Vibration minimiert.

Da, wie in Fig. 2 dargestellt ist, auch eine Bewegungsübertragung über die Welle 18 in gekrümmter Form erfolgen muß, muß zur Reibungsminimierung der Biegemodul der Welle 18 möglichst gering sein.

Die Schwungmasse, die vibrieren kann, muß möglichst gering sein. Die Abmessungen einer Asymmetrie an der Welle 18 sollen möglichst klein sein. Die relative Oberflächengeschwindigkeit zwischen der Welle 18 und der Kunststoffummantelung 28 muß möglichst gering sein. Die Fläche eines allfälligen Schmierfilms um die Welle 18 muß möglichst klein sein.

Der Durchmesser der Welle 18 geht somit mehrfach in die Reibungsverluste ein. Die Welle 18 wird wegen den obigen Punkten möglichst mit kleinem Durchmesser gewählt. Da der Anpreßdruck des Arbeitskopfes 14 der Werkzeugeinheit 12 meist klein ist, muß auch nur ein kleines Drehmoment übertragen werden. D.h. der Durchmesser der Welle 18 muß nur gerade so groß sein, daß es das Maximaldrehmoment des Motors 22 übertragen kann.

Die Möglichkeit zur Selbstbalancierung und die Vibrationsdämpfung des Werkzeugeinsatzes 10 erlauben problemlos hohe Drehzahlen, beispielsweise über 30.000 UpM. Zu dem ist durch die Miniaturisierung der gesamten Übertragungseinheit aus Werkzeugeinheit 12, Welle 18 und Kupplungselement 20 die erforderliche Motorleistung minimal, beispielsweise 2W, was die Verwendung eines kleinen Motors 22 und somit ein graziles Gerätedesign ermöglicht.

Die flexible Welle 18 kann statt aus einer Litze auch aus einem Profil, beispielsweise einem Draht, einem Rohr oder einem kantigen Profil bestehen, und insbesondere aus einer hochflexiblen Legierung wie Nitinol®, Tinel® hergestellt sein.

Anstelle eines strombetriebenen Motors 22 kann auch ein druckluftbetriebener Motor verwendet werden, und als Vibrationsdämpfung der Welle 18 kann ein Flüssigkeitsbad verwendet werden, in dem die Welle 18 rotiert.

In Fig. 1 ist in dem Arbeitsschaft 36 des Bearbeitungsgerätes neben dem Einsatzkanal 34 ein dazu paralleler Kanal 62 zur Führung eines Endoskops 56 vorhanden, von dem in Fig. 1a nur das distale Ende des Endoskopschaftes dargestellt ist. Das Endoskop 56 umfaßt eine nicht näher dargestellte Lichtzuführung, aus der distalseitig Beleuchtungslicht 58 austritt. Das Endoskop 56 umfaßt weiterhin eine Endoskopoptik, um einen Arbeitsbereich, der auch den Arbeitskopf 14 der Werkzeugeinheit 12 enthält, endoskopisch betrachten zu können. Mit dem Bezugszeichen 60 ist das Gesichtsfeld der Endoskopoptik dargestellt.

Mit Bezug auf Fig. 3 wird nachfolgend ein erstes Ausführungsbeispiel eines endoskopischen Bearbeitungsgerätes 70 beschrieben, das einen starren Arbeitsschaft 74 aufweist.

Das Bearbeitungsgerät 70 weist ein standardmäßiges starres Endoskop oder Boreskop 72 mit einer Blickrichtung von 0° bis etwa 30° auf, das in den Arbeitsschaft 74 eingeschoben und über einen Bajonettverschluß 84 verriegelt werden kann. Der Arbeitsschaft 74 enthält den Einsatzkanal 34 für den austauschbaren Werkzeugeinsatz 10 gemäß Fig. 1.

In dem austauschbaren Werkzeugeinsatz 10 sind die Verschleißteile wie Arbeitskopf 14 (anwendungsspezifisch), die Lagerbuchse 38 und die flexible Welle 18 zu einem vom Anwender einfach austauschbaren Zubehörteil zusammengefaßt.

In den Arbeitsschaft 74 können weitere universell einsetzbare Arbeitskanäle integriert sein. Diese können für Sonden (Faßzangen, angetriebene und ungetriebene Werkzeuge, Sensoren, Zusatzlichtfasern, UV-Analyselicht, UV-Aushärtungslicht, Laser-Bearbeitungsfasern, Faseroptik zur Beobachtung oder Spektroskopie, etc.) und /oder Medien (Klebstoff, Beschichtungen, Farbe, Chemikalien, Kühlmittel, Schmiermittel, Pulver, Druckluft, Gase, Wasser, etc.) verwendet werden. Auch die Absaugung von Material aus der Bearbeitungsgegend ist möglich (Schleifstoff, Verschmutzungen, Probenentnahmen zur Analyse).

Die vorgenannten Arbeitskanäle sind vorzugsweise mit einem genormten Kupplungsanschluß 78, beispielsweise einem Luer-Anschluß, versehen. Dort lassen sich neben Zuleitungen auch Sonden etc. verriegeln. Ein Ausblick 76 des Endoskops 72 kommt etwa an der Stirnseite am distalen Ende des Arbeitsschaftes 74 zu liegen. Proximal am Arbeitsschaft 74 ist ein Handgriffgehäuse 80, das den Antriebsmotor 22, Schalter 81, sowie Elektronik im Innern enthält. Das Handgriffgehäuse 80 dient ferner als Basis für die Befestigung und Durchführung der zuvor genannten Arbeitskanäle und eines Anschlußkabels 82 nach proximal. Das geringe Gewicht und die Form des Handgriffgehäuses 80 ermöglichen ein ergonomisches Halten. Die Länge des Handgriffgehäuses 80 wird aber zugunsten einer größeren Arbeitslänge möglichst kurz gehalten.

Das Anschlußkabel 82 wird mit einer multifunktionellen Versorgungseinheit 86 (vgl. Fig. 4) verbunden, die neben der Versorgungsenergie (primär Elektrizität, aber beispielsweise auch Druckluft möglich) für den Motor 22 auch beispielsweise das Beleuchtungslicht für das Endoskop 72 liefern kann.

Zum Betrieb wird Beleuchtungslicht von einer Lichtquelle durch einen Lichtanschluß 73 in das Endoskop 72 geleitet. Das am distalen Ende austretende Licht beleuchtet den Hohlraum und den Arbeitskopf 14. Das Bearbeitungsgerät 70 arbeitet mit hoher Tourenzahl, ähnlich einem Zahnarzt-Bohrer, was bei kleinen Auflagekräften große Abtragleistungen und exakte Führbarkeit ermöglicht.

Das Bearbeitungsgerät 70 kann entweder mit dem Schalter 81 im Handgriffgehäuse 80 oder mit einem Fußschalter aktiviert werden. Die Drehzahl kann vorzugsweise eingestellt werden, um sie der verwendeten Werkzeugeinheit 12 bzw. dem Arbeitskopf 14 und der Situation anzupassen.

Die Drehrichtung ist bei bestimmten flexiblen Wellen 18 in beide Richtungen möglich, so daß die Drehrichtung passend zu der Arbeitssituation gewählt werden kann. Dabei kann auch eine intermittierende und/oder wechselweise Rotation erfolgen, wenn es beispielsweise für eine bessere Präzision und/oder Abtragleistung vorteilhaft ist.

Das Bearbeitungsgerät 70 kann unter Sichtkontrolle durch das Endoskop 72 an die Bearbeitungsstelle geführt werden können, wo sofort mit der Bearbeitung begonnen werden kann.

Durch den modularen Aufbau läßt sich jederzeit das teure Endoskop 72 vom Arbeitsschaft 74 leicht trennen. Dies wird durch die einfache lösbare Verbindung mittels des universellen Bajonettverschlusses 84 ermöglicht. Das getrennt einsetzbare Endoskop 72 ermöglicht eine genauere Inspektion der zu bearbeitenden Stelle, da die Optik näher an die Oberfläche zur vergrößerten Detaildarstellung heran geführt werden kann, weil kein Arbeitskopf 14 eines Werkzeugeinsatzes 10 das Gerät auf Distanz hält.

In Fig. 4 ist ein weiteres Ausführungsbeispiel eines Bearbeitungsgerätes 90 dargestellt, das dem Bearbeitungsgerät 70 in Fig. 3 ähnlich ist, so daß nachfolgend nur die Unterschiede beschrieben werden.

Ein Arbeitsschaft 92 des Bearbeitungsgerätes 90 ist gegenüber dem Arbeitsschaft 74 des Bearbeitungsgerätes 70 mit einem flexiblen Teil bzw. einer Lenkung 94 ausgebildet, so daß die Werkzeugeinheit 12 mit dem Arbeitskopf 14 des Werkzeugeinsatzes 10 ausgelenkt werden kann. Vorzugsweise und wegen einer höheren mechanischen Stabilität geht die Ablenkung vorzugsweise in eine Richtung bis zu einem vordefinierten Anschlag, bei dessen Erreichen sich die Lenkung konstruktionsbedingt abrupt versteift. Diese Versteifung verbessert die präzise Führung der Werkzeugeinheit 12. Die Ablenkung in nur einer Richtung ermöglicht trotzdem eine Bearbeitung eines großen Bereichs in einem Hohlraum, da das Bearbeitungsgerät 90 auch einfach um seine Längsachse gedreht werden kann. Ein Endoskop 96 des Bearbeitungsgerätes 90 weist in diesem Ausführungsbeispiel im Unterschied zu dem Ausführungsbeispiel gemäß Fig. 3 eine Blickrichtung von c.a. 70° auf, wobei ein Ausblick 98 vor der Lenkung 94 durch eine entsprechende Öffnung im Mantel des Arbeitsschaftes 92 vorgesehen ist. Die Ablenkung des distalen Endes des Arbeitsschaftes 92 beträgt in diesem Ausführungsbeispiel ca. 90°. Die Abmessungen, die Blickrichtung und das Gesichtsfeld (beispielsweise 30°) sind so koordiniert, daß der Arbeitskopf 14 an geeigneter Stelle im Bild des Endoskops 96 zu liegen kommt, beispielsweise in der Bildmitte oder im unteren Bilddrittel, und so, daß eine angemessene Detailerkennung der zu bearbeitenden Oberfläche ermöglicht wird. Die Winkel für die Ablenkung und das Gesichtsfeld etc. sind anwendungsbedingt.

Die Ablenkung des distalen Endes des Arbeitsschaftes 92 wird vorzugsweise mit einem relativ kleinen gerändelten Drehknopf 100 am proximalen Ende eines Handgriffgehäuses 102 über einen vorzugsweisen bidirektional wirkenden Bowdenzug (nicht dargestellt) bewirkt. Im Inneren des Drehknopfes 100 befindet sich ein nicht näher dargestelltes selbsthemmendes Gewinde, das die Rotation des Drehknopfes 100 in eine Translation des Bowdenzuges übersetzt. Anschläge im Mechanismus des Drehknopfes 100 verhindern dabei ein Überdehnen des Bowdenzuges in beide Richtungen.

Das bewegliche Element des bidirektional wirkenden Bowdenzuges ist aus einer Litze und aus einer sie umgebenden dicht anliegenden Spirale aufgebaut, die an zumindest den beiden Enden fest miteinander verbunden sind. Der Zug wird dabei durch das Seil, der Schub durch die Spirale übertragen. Ein starres Rohr bildet die Außenhülle des Bowdenzuges zur Führung und Aufnahme der Gegenkraft.

Durch die Bidirektionalität des Zuges wird Querschnitt eingespart, da zum Strecken und Beugen des Distalendes nur ein Zug benötigt wird, der auf Zug und Schub belastet wird. Der bei ablenkbaren Endoskopen übliche Gegenzug entfällt. Durch das fehlen des Elementes des Gegenzuges erhält man mehr Freiheit für die Wahl und Verteilung der anderen Elemente im Innern des Arbeitsschaftes 92. Ferner kann der Drehpunkt der Ablenkglieder asymmetrisch von der Mitte an den dem Zug gegenüberliegenden Ort verlegt werden, was für die Ablenkung eine größere Hebelwirkung und somit eine größere Ablenkkraft bewirkt. In dem Arbeitsschaft 92 ist zumindest ein Arbeitskanal bzw. ein Einsatzkanal vorgesehen, wie mit Bezug auf Fig. 3 bzw. auf Fig. 1 beschrieben ist.

Das Bearbeitungsgerät 90 wird in geradem Zustand bis zur ungefähren Arbeitsposition eingeführt. Hier kann eine eingeschobene Faserbildleitersonde eine Einführung unter Sichtkontrolle ermöglichen, da das Endoskop 96 nicht für einen Geradeausblick vorgesehen ist. Nach Erreichen der ungefähren Arbeitsposition wird das distale Ende des Arbeitsschaftes 92 mit dem Drehknopf 100 in Arbeitsstellung gebracht, wobei am Ende des Anschlags die vorteilhafte Versteifung eintritt. Eine Variante der Lenkung 94 sieht auch einen Mechanismus vor, der durch ein weiteres Betätigungselement eine Versteifung des flexiblen Teils 94 in beliebiger Ablenkposition ermöglicht, ohne daß die Ablenkung bis zum Anschlag ausgelenkt werden muß.

Wenn die richtige Ablenkposition eingestellt ist, kann der Motor aktiviert werden und die endoskopische Bearbeitung der Oberfläche unter gleichzeitiger Beobachtung ausgeführt werden.

Das Bearbeitungsgerät 90 wird vorteilhafterweise wieder mit gerade gerichteter Ablenkung aus dem Bearbeitungsraum entfernt.

Analog zu der Ausführung gemäß Fig. 3 kann auch hier das eingesetzte Endoskop 96 aus dem Arbeitsschaft 92 entnommen werden, um eine vergrößerte Detailbetrachtung der Oberfläche durch Benutzung des Endoskops 96 allein zu ermöglichen.

Eine Modifikation des in Fig. 4 gezeigten Ausführungsbeispiels besteht darin, kein starres Endoskop 96 einzusetzen, sondern statt dessen eine Faseroptik zu verwenden, die an der Stirnseite des distalen Endes des Arbeitsschaftes 92 endet, ähnlich wie in Fig. 3. Der Ablenkmechanismus und der zusätzliche Arbeitskanal ist wiederum wie zuvor mit Bezug auf Fig. 4 beschrieben ausgebildet. Mit dem erfindungsgemäßen Bearbeitungsgerät gemäß den zuvor genannten Ausführungsbeispielen kann eine große Vielfalt an Oberflächengeometrien bearbeitet werden.

## Patentansprüche

1. Austauschbarer Werkzeugeinsatz für ein endoskopisches Bearbeitungsgerät (70; 90), mit einer Welle (18), die an ihrem distalen Ende mit einer Werkzeugeinheit (12) und an ihrem proximalen Ende mit einem Kupplungselement (20) zur lösbaren Verbindung mit einem Motor (22) verbunden ist, wobei die Welle (18) flexibel als Litze oder als Profil in Form eines Drahtes, Rohrs oder kantigen Profils ausgebildet ist, und mit einem Verriegelungsmechanismus (44) zum lösbaren Halten des Werkzeugeinsatzes (10) am Bearbeitungsgerät (70; 90) **dadurch gekennzeichnet, daß** der Verriegelungsmechanismus (44) ein an der Werkzeugeinheit (12) angeordnetes Verriegelungselement (52) aufweist, in das ein Verriegelungsteil (50) des Bearbeitungsgerätes (70; 90) eingreift, und daß die Welle (18) so mit der Werkzeugeinheit (12) verbunden ist, daß diese eine Einheit bilden, die bei Verriegelung des Verriegelungsmechanismus (44) lösbar am Bearbeitungsgerät (70; 90) gehalten wird und nach Entriegelung des Verriegelungsmechanismus (44) als Ganzes entnommen werden kann.

2. Werkzeugeinsatz nach Anspruch 1, **dadurch gekennzeichnet, daß** die Welle (18) auch mit dem proximalen Kupplungselement (20) so verbunden ist, daß das Kupplungselement (20) zusammen mit der Welle (18) und der Werkzeugeinheit (12) nach Lösen des Verriegelungsmechanismus entnommen werden kann.

3. Werkzeugeinsatz nach Anspruch 1 oder 2, **dadurch gekennzeichnet, daß** die Werkzeugeinheit (12) einen Arbeitskopf (14) und einen Werkzeugschaft (16) aufweist, und daß das Verriegelungselement (52) als umlaufende Nut in dem Werkzeugschaft (16) ausgebildet ist.

4. Werkzeugeinsatz nach Anspruch 3, **dadurch gekennzeichnet, daß** eine Lagerbuchse (38) vorgesehen ist, die einen Längsabschnitt des Werkzeugschafts (16) aufnimmt und einen Schlitz (53) aufweist, der so ausgebildet ist, daß das Verriegelungselement (52) den Schlitz (53) durchgreifen kann.

5. Werkzeugeinsatz nach einem der vorhergehenden Ansprüche, **dadurch gekennzeichnet, daß** die Welle (18) aus einer hochflexiblen Legierung gebildet ist.

6. Werkzeugeinsatz nach einem der vorhergehenden Ansprüche, **dadurch gekennzeichnet, daß** eine Spirale (26) vorgesehen ist, die einen Längsabschnitt der Welle (18) in der Spirale (26) drehbar aufnimmt.

7. Werkzeugeinsatz nach Anspruch 6, **dadurch gekennzeichnet, daß** eine Ummantelung, vorzugsweise eine Kunststoffummantelung (28) vorgesehen ist, die die Spirale (26) umgibt.

8. Endoskopisches Bearbeitungsgerät, **gekennzeichnet durch** einen Werkzeugeinsatz (10) nach einem der Ansprüche 1 bis 7.

9. Bearbeitungsgerät nach Anspruch 8, **dadurch gekennzeichnet, daß** sich daß Verriegelungsteil (50) senkrecht zur Längsachse des Bearbeitungsgerätes (70; 90) erstreckt und an seinem der Längsachse abgewandten Ende an einem parallel zur Längsachse verlaufenden Halteteil des Verriegelungsmechanismus (44) angebracht ist.

10. Bearbeitungsgerät nach Anspruch 9, **dadurch gekennzeichnet, daß** das Halteteil als im Wesentlichen senkrecht zur Längsachse federnde Lasche (48) ausgebildet ist, so daß das Verriegelungsteil (50) senkrecht zur Längsachse bewegbar ist.

11. Bearbeitungsgerät nach Anspruch 10, **dadurch gekennzeichnet, daß** der Verriegelungsmechanismus (44) eine in axialer Richtung verlagerbare Schiebehülse (46) aufweist, die über die Lasche (48) schiebbar ausgebildet ist und das Verriegelungsteil (50) senkrecht zur Längsachse bewegt, so daß das Verriegelungsteil (50) in Eingriff mit dem Verriegelungselement (52) des Werkzeugeinsatzes (10) bringbar ist.

12. Bearbeitungsgerät nach einem der Ansprüche 8 bis 11, **dadurch gekennzeichnet, daß** ein Einsatzkanal (34) vorgesehen ist, in den der Werkzeugeinsatz (10) in axialer Richtung ein- und ausführbar ist, wobei der Werkzeugeinsatz (10) in dem Einsatzkanal (34) mit radialem Spiel aufgenommen ist.

13. Bearbeitungsgerät nach Anspruch 12, **gekennzeichnet durch** einen Arbeitsschaft (36; 74; 92), in dem der Einsatzkanal (34) und parallel zu diesem ein Kanal für ein Endoskop (56; 72; 96) angeordnet ist.

14. Bearbeitungsgerät nach Anspruch 13, **dadurch gekennzeichnet, daß** der Arbeitsschaft (92) im Distalbereich ein flexibles Teil (94) zum Auslenken der Werkzeugeinheit (12) aus der Längsachse des Proximalbereichs des Arbeitsschafts (92) aufweist.

## Claims

1. Exchangeable tool assembly for an endoscopic treatment device (70; 90), comprising a shaft (18) connected at its distal end to a tool unit (12) and at its proximal end to a coupling element (20) for releasably connecting to a motor (22), wherein the shaft (18) is configured as a braided cable or as a profile in form of a wire, a tube or an angular profile so as to be flexible, and a locking mechanism (44) for releasably holding the tool unit (10) on the treatment device (70; 90), **characterized in that** the locking mechanism (44) comprises a locking element (52) arranged at the tool unit (12), in which a locking part (50) of the treatment device (70; 90) engages, and that the shaft (18) is connected with the tool unit (12) such that these form a unit which is releasably held on the treatment device (70; 90) when the locking mechanism (44) is locked, and can be removed as a whole after unlocking the locking mechanism (44).

2. The tool assembly of claim 1, **characterized in that** the shaft (18) is likewise connected with the proximal coupling element (20) in such a way that the coupling element (20) can be jointly removed with the shaft (18) and the tool unit (12) after releasing the locking mechanism.

3. The tool assembly of claim 1 or 2, **characterized in that** the tool unit (12) comprises a working head (14) and a tool shank (16), and that the locking element (52) is configured as an annular groove in the tool shank (16).

4. The tool assembly of claim 3, **characterized in that** a bearing bush (38) is provided which receives a longitudinal portion of the tool shank (16) and has a slot (53) which is configured such that the locking element (52) can pass through the slot (53).

5. The tool assembly of anyone of the preceding claims, **characterized in that** the shank (18) is made from a highly flexible alloy.

6. The tool assembly of anyone of the preceding claims, **characterized in that** a spiral (26) is provided, which rotatably receives a longitudinal portion of the shank (18) in the spiral (26).

7. The tool assembly of claim 6, **characterized in that** a mantel, preferably a plastic mantel (28) is provided surrounding the spiral (26).

8. Endoscopic treatment device, **characterized by** a tool assembly (10) according to anyone of claims 1 through 7.

9. The treatment device of claim 8, **characterized in that** the locking part (50) extends perpendicularly to the longitudinal axis of the treatment device (70; 90) and at its end opposite to the longitudinal axis is attached to a holding part of the locking mechanism (44) extending parallel to the longitudinal axis.

10. The treatment device of claim 9, **characterized in that** the holding part is configured as a bracket (48) which is resilient in the direction substantially perpendicular to the longitudinal axis so that the locking part (50) is movable in direction perpendicular to the longitudinal axis.

11. The treatment device of claim 10, **characterized in that** the locking mechanism (44) comprises a slide sleeve (46) displaceable in axial direction, which is configured such that it can be slid over the bracket (48) and moves the locking part (50) perpendicularly to the longitudinal axis so that the locking part (50) can be engaged with the locking element (52) of the tool assembly (10).

12. The treatment device of anyone of claims 8 through 11, **characterized in that** an inset channel (34) is provided, into which the tool assembly (10) can be introduced or from which the tool assembly (10) can be withdrawn in axial direction, wherein the tool assembly (10) is received in the inset channel (34) with radial play.

13. The treatment device of claim 12, **characterized by** a working shank (36; 74; 92), in which the inset channel (34) and, parallel thereto, a channel for an endoscope (56; 72; 96) is arranged.

14. The treatment device of claim 13, **characterized in that** the working shank (92), in the distal region, comprises a flexible part (94) for deflecting the tool unit (12) from the longitudinal axis of the proximal region of the working shank (92).

## Revendications

1. Elément d'outil amovible pour un dispositif d'usinage (70 ; 90) endoscopique, comportant un arbre (18), dont l'extrémité distale est reliée à une unité d'outil (12) et dont l'extrémité proximale est reliée à un élément de couplage (20) pour un assemblage amovible avec un moteur (22), l'arbre (18) étant réalisé de manière flexible sous forme de toron ou de profilé sous forme d'un câble, d'un tube ou d'un profilé polygonal, et comportant un mécanisme de verrouillage (44) pour le soutient amovible de l'élément d'outil (10) sur le dispositif d'usinage (70 ; 90), **caractérisé en ce que** le mécanisme de verrouillage (44) comporte un élément de verrouillage (52), qui est agencé sur l'unité d'outil (12) et dans lequel s'engage une pièce de verrouillage (50) du dispositif d'usinage (70 ; 90), et **en ce que** l'arbre (18) est relié à l'unité d'outil (12) de telle sorte qu'ils forment une unité qui, lors du verrouillage du mécanisme de verrouillage (44), est soutenue de manière amovible contre le dispositif d'usinage (70 ; 90) et peut être retirée dans son ensemble après le déverrouillage du mécanisme de verrouillage (44).

2. Elément d'outil selon la revendication 1, **caractérisé en ce que** l'arbre (18) est également relié à l'élément de couplage (20) proximal de telle sorte que l'élément de couplage (20) peut être retiré, conjointement avec l'arbre (18) et l'unité d'outil (12), après la désolidarisation du mécanisme de verrouillage.

3. Elément d'outil selon la revendication 1 ou 2, **caractérisé en ce que** l'unité d'outil (12) comporte une tête de travail (14) et une tige d'outil (16), et **en ce que** l'élément de verrouillage (52) est réalisé sous forme de rainure périphérique dans la tige d'outil (16).

4. Elément d'outil selon la revendication 3, **caractérisé en ce qu'**il est prévu un coussinet (38), qui reçoit un tronçon longitudinal de la tige d'outil (16) et comporte une fente (53) qui est réalisée de telle sorte que l'élément de verrouillage (52) peut engager la fente (53).

5. Elément d'outil selon l'une quelconque des revendications précédentes, **caractérisé en ce que** l'arbre (18) est réalisé dans un alliage à haute flexibilité.

6. Elément d'outil selon l'une quelconque des revendications précédentes, **caractérisé en ce qu'**il est prévu une spirale (26), dans laquelle un tronçon longitudinal de l'arbre (18) est logé de manière à pouvoir tourner dans la spirale (26).

7. Elément d'outil selon la revendication 6, **caractérisé en ce qu'**il est prévu une enveloppe, de préférence une enveloppe en matière plastique (28), qui entoure la spirale (26).

8. Dispositif d'usinage endoscopique, **caractérisé par** un élément d'outil (10) selon l'une quelconque des revendications 1 à 7.

9. Dispositif d'usinage selon la revendication 8, **caractérisé en ce que** la pièce de verrouillage (50) s'étend perpendiculairement à l'axe longitudinal du dispositif d'usinage (70 ; 90) et est montée, au niveau de son extrémité opposée à l'axe longitudinal, contre une pièce support du mécanisme de verrouillage (44), laquelle est parallèle à l'axe longitudinal.

10. Dispositif d'usinage selon la revendication 9, **caractérisé en ce que** la pièce support est réalisée sous forme de patte (48) flexible sensiblement perpendiculairement à l'axe longitudinal, de telle sorte que la pièce de verrouillage (50) est mobile perpendiculairement à l'axe longitudinal.

11. Dispositif d'usinage selon la revendication 10, **caractérisé en ce que** le mécanisme de verrouillage (44) comporte un manchon coulissant (46) mobile dans le sens axial, lequel est réalisé de manière à pouvoir coulisser au-dessus de la patte (48) et déplace la pièce de verrouillage (50) perpendiculairement à l'axe longitudinal, de telle sorte que la pièce de verrouillage (50) peut être amenée en prise avec l'élément de verrouillage (52) de l'élément d'outil (10).

12. Dispositif d'usinage selon l'une quelconque des revendications 8 à 11, **caractérisé en ce qu'**il est prévu un conduit d'outil (34), dans lequel l'élément d'outil (10) peut être introduit et extrait dans le sens axial, l'outil rapporté (10) étant logé avec un jeu radial dans le conduit d'outil (34).

13. Dispositif d'usinage selon la revendication 12, **caractérisé par** une tige de travail (36 ; 74 ; 92), dans laquelle sont agencés le conduit d'outil (34) et, parallèlement à celui-ci, un conduit pour un endoscope (56 ; 72 ; 96).

14. Dispositif d'usinage selon la revendication 13, **caractérisé en ce que** la tige de travail (92) comporte, dans la zone distale, une partie flexible (94) pour dévier l'unité d'outil (12) hors de l'axe longitudinal de la zone proximale de la tige de travail (92).
